# EUROPEAN PATENT SPECIFICATION

(11) **EP 0 745 433 B1**
(45) Date of publication and mention of the grant of the patent: **27.06.2001**
(21) Application number: 95108433.4
(22) Date of filing: 01.06.1995
(51) Int. Cl.: B05C 1/16, A61F 13/15

(54) **Adhesive printing for disposable absorbent article**
Verfahren zum Bedrucken eines Klebstoffs auf einen absorbierenden Artikel
Procédé d'impression d'un adhésif sur un produit absorbant

(43) Date of publication of application: 04.12.1996
(73) Proprietor: THE PROCTER & GAMBLE COMPANY, Cincinnati, Ohio 45202 (US)
(72) Inventor: Hagemeister, Eric Peter, 74532 Ilshofen (DE); Hundorf, Harald Hermann, 61352 Bad Homburg (DE); Lender, Horst Andreas, 91740 Röckingen (DE); Plumley, Julian Ashton, 74589 Satteldorf (DE)
(74) Representative: Hirsch, Uwe Thomas

(56) References cited:
- EP-A- 0 183 460
- EP-A- 0 539 032
- EP-A- 0 611 607
- EP-A- 0 621 082
- GB-A- 2 134 420
- US-A- 5 259 902

## Description

### Field of the invention

The present invention relates to a process for providing a disposable absorbent article according to the precharacterising part of claim 1. A process of this type is known from e.g. US-A-5 064 492. Disposable absorbent articles are such as sanitary napkins, panty liners, catamenials, incontinence inserts, and diapers for adults or babies.

### Background of the invention

Absorbent articles such as sanitary napkins, panty liners, catamenials, incontinence inserts and diapers for adults or babies are commonly provided with an adhesive on their garment-facing surface to attach them during their usage period to a garment of the user. In particular sanitary napkins and panty liners are commonly provided with a pressure sensitive, hotmelt, adhesive which attaches to the undergarment of the wearer and thereby improves fit and comfort of the product for the wearer. These adhesives are typically covered with a release paper prior to use.

More generally absorbent articles are provided with adhesive areas in order to combine materials which ultimately make up part or the whole of the absorbent article. In particular, multi-layer structures forming the topsheet, core or backsheet are often combined by adhesives. The combining of the topsheet, the core and the backsheet to each other also can be accomplished by adhesives.

Typically these products are made by high speed machinery. The machinery includes equipment which adds the adhesive in a very fast and efficient manner, ensuring consistency of the absorbent products over large quantities thereof.

A common way of providing an adhesive is a slot-coating or spraying of the adhesive onto a continuously conveyed thread of material, as shown for example in US 5 259 902. The surface of this material, coated with adhesive, is then joined to another material forming all or part of the disposable absorbent articles. These adhesive application methods allow adhesive application without contact between apparatus and material to be coated. This is desirable in particular for thermoplastic film or nonwoven materials often used in disposable absorbent articles which are very heat sensitive and can be incompatible with adhesives which require high melting temperatures.

An alternative method of adhesive application to material used in absorbent articles is a screen printing method. In the screen printing method an adhesive is applied to the inside of a rotating roll having apertures. This roll, also called screen, is contacted with the continuous conveyed threat of material such that adhesive is transferred through the holes of the material. Again, only the adhesive is heated while the screen surface contacting the surface to which the adhesive is applied is not heated. A Similar method is disclosed for example in GB 2 134 420 whereby the apertured roll is partly immersed in an adhesive container.

Another alternative application method for adhesive is to render one of the materials used in the production of absorbent articles adhesive. This can be done in a separate step independent and long before use of the material in the article. For example adhesive tape or film - as used on baby diapers - have one adhesive surface and one non-adhesive surface. The adhesive surface is not firmly attached (or at least releasably attached) to the non-adhesive surface by winding the tape or film up into a roll. Upon use of the material the roll is then unwound to present the adhesive surface to the material to which it is intended to attach the tape or film.

US 5,064,492 provides in this manner an impermeable film onto which a particular adhesive, which is said to be non-blocking below about 43°C (110°F), is printed. The adhesive is provided to the film by a patterned printing roll and a cooled counter roll or a series of such printing units if patterns in the adhesive are desired. The winding of the adhesive coated film is conducted below the blocking temperature.

This method of providing precoated film adds multiple complexities to logistics, storage and final manufacturing of disposable articles. Even accidental heating of any portion of the precoated roll would render it useless (sticking to itself). Also all benefits of the positioning accuracy of adhesive printing are lost in the alignment difficulties associated with film unwinding. Further the adhesive has to be heated on the film to return it to a state of stickiness such that added energy consumption is paired with a probability of melting through the underlying film material. Also this heating of the adhesive causes degradation due to the added heating/cooling cycle.

One common drawback of all the above mentioned adhesive application processes is their inflexibility, inaccuracy relative to the shape of the adhesive to be applied and that they essentially can only provide the adhesive continuously. On/Off systems for coating or spraying have the drawback of delay in their systems response and generally the problems associated with accelerating and decelerating mass streams. For hollow drum screen printing it is possible to create a pattern in the sereen which would allow to create adhesive patterns. However, this is limited in that the screen has to provide a fairly even distribution of adhesive application sites (holes) in order to exclude temperature variations resulting in unstable application processes. Also adhesive screen printing cannot provide an even, full surface adhesive coverage due to the maximum apertured dimensions and total open area of such a screen in respect to its stability.

The applicants have now found a process of providing adhesive areas to disposable absorbent articles, which areas are not limited in their shape. The process allows reduced adhesive area or full surface adhesive coverage, and is generally more stable and accurate than prior art processes. The inventive process causes less production down time than a process providing the same adhesive but using conventional adhesive delivery methods.

It is therefore an object of the invention to provide disposable absorbent articles having an adhesive area which joins two materials comprised in the disposable absorbent article to provide an accurate and efficient process alternative to current adhesive applications thereby allowing adhesive area shape designs so far not possible at the production speeds typical for disposable absorbent articles and to reduce adhesive consumption by better control of the adhesive area placement and adhesive quantity variation.

### Summary of the invention

The above object is achieved according to the invention by a process as defined in claim 1. Particular embodiments of the invention are the subject of the dependent claims.

The present invention relates to a process for providing a disposable absorbent article comprising a first material and a second material which are joined by adhesive. The first material comprises a first surface and the second material comprises a second surface. The process comprises the steps of providing and conveying the first material in a machine direction, providing and conveying the second material, providing an adhesive area by roll printing onto at least part of the first surface and interfacing the adhesive area with the second surface in order to join both materials.

The roll printing step of the process according to the present invention is preferably conducted by a rotating transport roll for transporting the adhesive. The transport roll receives the adhesive in a receiving portion of its rotation path. The transport roll delivers the adhesive to the first surface in the delivering portion of the rotation path of the transport roll. The adhesive is typically a hot melt adhesive which is solid or in a semisolid plastic state at temperatures below the usage temperature of the disposable absorbent product in which the adhesive is utilized. The adhesive is applied in a liquefied state typically achieved by melting. Therefore, the receiving portion of the rotation path of the transport roll is preferably inside a bath of molten adhesive, even so other application means to the roll such as spraying could be used.

In a preferred embodiment of the present process the adhesive is provided to the first surface from below. This ensures drip free adhesive application without the need for additional safeguards to prevent dripping.

It is preferred that the process according to the present invention provides the adhesive area in a shape which comprises parts which are neither parallel nor perpendicular to the machine direction and which are preferably non-linear.

If so desired the adhesive area provided on the first surface has a peripheral edge which at least partially coincides with the peripheral edge of the surface of the material to which the adhesive area is provided. Preferably the adhesive area is provided to fully cover the surface of the first material such that their peripheral edges fully coincide.

The adhesive area can provide a releasable attachment means such as is typical between a release paper and a panty fastening adhesive. However, the current process is not limited to such adhesive application but can also provides a permanent connection between the first and the second material.

### Brief description of the drawings

Figure 1 shows a schematic view of an embodiment of the process steps of the present invention.

Figure 2 shows an alternative embodiment to figure 1 with the adhesive printing located differently.

Figure 3 shows a cross-sectional schematic view of the printing equipment used in the adhesive printing step according to the present invention.

Figures 4 - 15 show alternative engravings and their cross-sectional topographies useful for the rotating transport roll according to the present invention.

### Detailed description of the invention

The absorbent article has a body facing surface, typically provided by a liquid permeable substrate of fibrous or film like structure often termed topsheet; a garment facing surface, preferably provided by a liquid impermeable, but breathable substrate often termed backsheet and an absorbent structure placed between the body facing surface and the garment facing surface, typically termed the absorbent core. The absorbent article has a longitudinal axis and a lateral axis and can comprise any of the components or features usual in the art. In particular side wrapping elements, side flap components, or wings as well as any sort of extensibility or elastication feature can be comprised in absorbent articles.

The disposable article for absorbing liquid is described below by reference to a sanitary napkin or panty liner. However products such as adult or baby diaper inserts comprising adhesives can similarly benefit from the process of the present invention.

A preferred sanitary napkin or panty liner made according to the present invention has a pair of side wrapping elements or "undergarment covering components". They provide coverage of the wearer's panties to reduce side soiling (i.e., staining of the edges of the panty crotch) and are typically smaller than conventional flaps or wings.

The function of side wrapping elements, whether integral or joined to the article after being formed separately, is further improved by rendering them extensible in one or both directions parallel to the longitudinal axis and/or lateral axis. The extensibility can be provided across all or only part of the side wrapping elements and can be achieved by pleating or ring-rolling those parts which are to be rendered extensible.

A typical sanitary napkin or panty liner comprises a liquid pervious topsheet, a liquid impervious backsheet joined to the topsheet, and an absorbent core positioned between the topsheet and the backsheet. On the backsheet is provided an adhesive area providing the panty-fastening adhesive which is covered by a release paper, wrapper or the like prior to use of the article.

The present invention relates to a process for providing a disposable absorbent article comprising a first material and a second material which are joined by adhesive. The materials may be any of the materials typical in the context of disposable absorbent articles. It includes permanent and releasable adhesive surfaces which need to be provided and therefore includes absorbent articles having an adhesive area on their garment facing surface which is covered by a protective cover means such as a release paper.

In the production of absorbent articles several adhesive connections are typically formed all of which, some of which or at least one of which utilises the process according to the present invention. Therefore, disposable absorbent articles generally suitable to benefit from application of the present invention are described below.

The adhesive applied by the process according to the present invention can be any of a series of adhesives. Typically, hot melt adhesives, typically comprising a thermo-plastic base material, in combination with a tackyfying resin, or a mixture of various such materials, can be applied by the present process. Typical hot melt adhesives have a minimum melting temperature of about 80°C, often even about 100°C. The requirement for these hot melt adhesives is of course that they maintain their adhesive performance until disposal of the disposable absorbent article, i.e. during manufacturing, storage, transport and use of the disposable absorbent article. Typically, the highest temperature during these activities is the usage temperature at about 40°C when the disposable absorbent article is used on the body of a human. However, higher temperatures can occur for example when articles are left in a vehicle in the sun, where temperatures of 60°C and higher have been reported.

When applying the adhesive by roll printing according to the present invention it is necessary that the cohesive forces of the adhesive are lower than the adhesive forces to the substrate to which the adhesive is printed and that the cohesive forces within the substrate onto which the adhesive is printed are also higher than the cohesive forces of the adhesive. If this force relation is not met parts of the substrate will disassociate from the substrate and attach to the adhesive surface on the roll printing roll. In order to assure this cannot happen it can be necessary to keep a tight temperature control since the cohesive strength of hot melt adhesives is highly dependent on the temperature of the adhesive. It is understood by those skilled in the art that simple trials will allow to immediately confirm whether the adhesive roll printing to a certain substrate can be performed and at which temperature given a certain adhesive.

In order to more fully assess the utility of the process of the present invention a description of a typical disposable absorbent article follows.

### Topsheet

The topsheet is compliant, soft feeling, and non-irritating to the wearer's skin. Useful topsheets are well known in the art and include especially those disclosed in copending application "Disposable absorbent articles having a shaped panty fastening adhesive."

When referring to the topsheet a multi layer structure or a mono layer structure is contemplated. Joining the topsheet to another portion of the absorbent article or internal joining of multi layer topsheets can be subject to the process of the present invention.

### Absorbent structure

When referring to the absorbent structure a multi layer structure or a mono layer structure are contemplated. Joining the absorbent structure to another portion of the absorbent article or internal joining of multi layer absorbent structures can be subject to the process of the present invention. Useful absorbent structures are well-known in the art and include especially those disclosed in copending application "Disposable absorbent articles having a shaped panty fastening adhesive".

For joining the surface of the absorbent structure or a layer of the absorbent structure to another surface it is important to observe the above remarks in respect to the cohesive and adhesive forces and their relation to each other. In particular absorbent structures of cellulosic fluff material have often been found to not satisfy the requirement of sufficient cohesive strength to allow printing to them. A possible solution in these circumstances is to use the adhesive roll printing process on the surface to which the cellulosic fibrous absorbent structure or layer of the absorbent structure is to be joined.

### Backsheet

The backsheet primarily prevents the exudates absorbed and contained in the absorbent structure from wetting articles that contact the absorbent product such as underpants, pants, pyjamas and undergarments. Useful backsheets are well known in the art and include especially those disclosed in copending application "Disposable absorbent articles having a shaped panty fastening adhesive". When referring to the backsheet a multi layer structure or a mono layer structure is contemplated. Joining the backsheet to another portion of the absorbent article or internal joining of multi layer backsheets can be subject process of the present invention.

### The panty-fastening-adhesive

The backsheet typically forms the garment facing surface of the absorbent article on which the panty fastening adhesive is placed. Panty-fastening-adhesives can comprise any adhesive or glue used in the art for such purposes. These adhesives typically are pressure sensitive and remain tacky well below their application temperature.

Suitable non-extensible adhesives are Savare LA203 and Savare LA303 made by Savare I.C. of Milan in Italy, Coramelt 867 by Koemmerling in Pirmasens in Germany, Fuller D3964ZP and Fuller H-2238ZP manufactured by the H.B. Fuller Co. in Lueneburg, in Germany. Suitable adhesive fasteners are also described in U.S. Patent 4,917,697.

It is a feature of the present invention that the shape of the panty-fastening adhesive area can comprise non-linear portions which are not coincident with the periphery of the garment facing surface of the absorbent article. The panty-fastening adhesive also need not but can be coextensive with the garment facing surface of the sanitary napkin.

Prior to use of the absorbent article the panty fastening adhesive is typically protected from contamination and from sticking to any surface where this is not desired by a protective cover means such as a silicone coated release paper, a plastic film or any other easily removable cover. The protective cover means can be provided as a single piece or in a multitude of pieces e.g. to cover individual adhesive areas. It also can perform other functions such as provide individualised packaging for the article or provide a disposal function.

### Process steps of the present invention

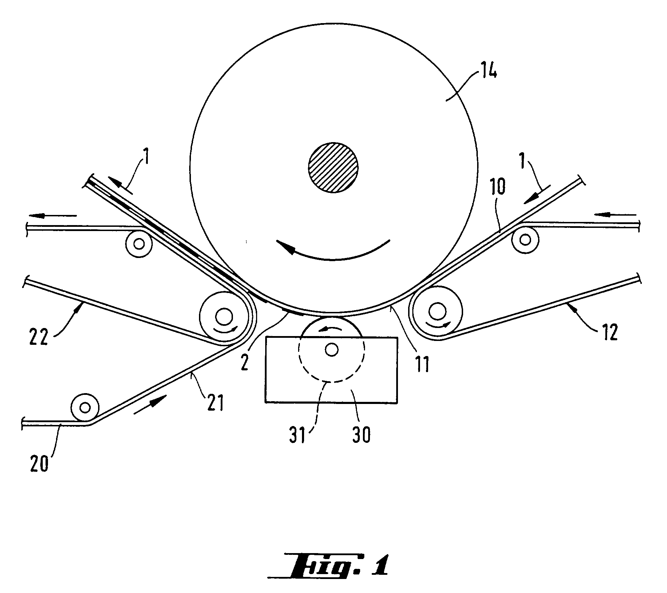

In the following the process according to the present invention will be described with reference to the drawings. In figure 1, a process according to the present invention is shown wherein a first material (10) is provided and conveyed in the machine direction designated 1 following the transport path of the first material (10). A second material (20) is also provided and conveyed. Figure 1 and figure 2 further show a schematic roll printing unit (30) where adhesive (2) is provided to the first surface (11) of the first material (10). In figure 1 and the detailed cross-sectional figure 3 the adhesive (2) is provided to the first surface (11) in an intermittent fashion. Figure 2 shows a continuous delivery of adhesive (2) to the first surface (11) of the first material (10). In figure 2 the roll printing unit (30) is also provided at a different location than in figure 1 and is below but not directly under the first material (10) in a gravitation sense.

Roll printing unit (30) is preferably directly under the material guiding system (14). The term "directly under" in this context is meant in a more restrictive sense then below in that the normal vector on the surface of material in the receiving section is parallel to the gravitational vector (40) but with an opposite direction.

Turning to figure 3 it can be seen that a first material (10) is conveyed in a machine direction (1) generally from the right hand side of the drawing guided by a material guiding system (12). From this guiding system the first material (10) is transferred to another material guiding system (14) conveying the material (10) towards the roll printing unit (30).

The roll printing unit (30) comprises a rotating transport roll (31). The circular cross-section of the surface of rotating transport roll (31) describes the rotating path of rotating transport roll (31). Roll (31) in the receiving portion of the rotating path (34) dives into an adhesive bath (36). This is typically a molten adhesive bath maintained inside the roll printing unit (30) at constant temperature by heating elements (not shown). Also roll (31) can and preferably does have internal heating elements such as hot oil or water channels to maintain the surface of transport roll (31) at a constant temperature.

The transport roll (31) is wetted by the molten adhesive in the receiving portion of rotating path (34). The roll surface continuously moves along the rotating path out of the bath of molten adhesive (36) and all excess adhesive is removed from the rotating transport roll (31) by scraper blade (35). Scraper blade (35) essentially rides on the surface of rotating transport roll (31). It can be kept at a constant nip by distance rings or other spacing mechanisms. It also can be provided with constant pressure onto the roll by spring loading scraper plate (35) or other means well known in the art of printing. Alternatively, the amount of adhesive taken up by rotating transport roll (31) can be controlled through the temperature and viscosity of the adhesive in the bath of molten adhesive (36) and the length of the receiving portion of the rotating path (34). Then a scraper plate (35) may not be necessary at all.

Following the path of the rotating transport roll (31) the delivery portion of the rotating path (33) is reached where the adhesive (2) is transferred from the transport roll to the first surface (11) of the first material (10). In order for the adhesive (2) to transfer it is necessary that the adhesive strength between the adhesive (2) and the first surface (11) exceeds either the cohesive strength of the adhesive or the adhesive strength between the adhesive (2) and the surface of the transport roll (31) or both. Once the adhesive (2) is transferred to the first surface (11) of the first material (10) it is further conveyed together with material (10) to be joined with the second material (20).

Material guiding system (14) provides the support for the first material (10) during printing such that a printing pressure above the breaking pressure of the first material can be used. If first material (10) can withstand the printing pressure without support then guiding system (14) is not absolutely necessary but desirable for printing placement accuracy.

Material (20) is provided and introduced into the process according to the present invention by guidance system (22) as shown in figure 3. The distance between the point of joining second material (20) with second surface (21) to first material (10) by interfacing the adhesive (2) between first surface (11) and second surface (21) needs to be carefully set in order to ensure that the adhesive is still in a state to provide the desired adhesive attachment between both materials. If the combined material does not already represent the final disposable absorbent article it is then further conveyed to additional process steps to ultimately provide a disposable absorbent article.

The pattern of the adhesive provided to the first surface of the first material can be defined by the printing surface of the rotating transport roll (31). For example in figure 3 engravings or gravertures (32) can be provided in any desired pattern. The whole transport roll surface, including the pattern of engravings (32), will be wetted by the adhesive (2) in the receiving part of the rotating path of the transport roll (31). At the scraper plate (35) adhesive clinging to the roll surface outside the engravings (32) is removed and guided back into the bath of molten adhesive (36). Only the engravings remain filled with the adhesive which is transported on to the delivering portion of the rotation path of the rotating transport roll.

The "pattern of engravings" as used herein refers to the macroscopic area which is to be provided as adhesive area onto the first surface (11). This pattern is formed of a multitude of engravings (32) which are typically small enough to provide the whole area of adhesive with a layer of adhesive of about constant quantity per surface area. For example, typical patterns of engravings to provide panty fastening adhesive are those shown in parallel patent application entitled "Disposable absorbent articles having a shaped panty fastening adhesive". Other adhesive applications may require other patterns such a peripheral edge pattern to join the backsheet to the topsheet around the periphery of an absorbent core. The number of alternatives is unlimited but suitable patterns for the application will typically be easily identified by those skilled in the art.

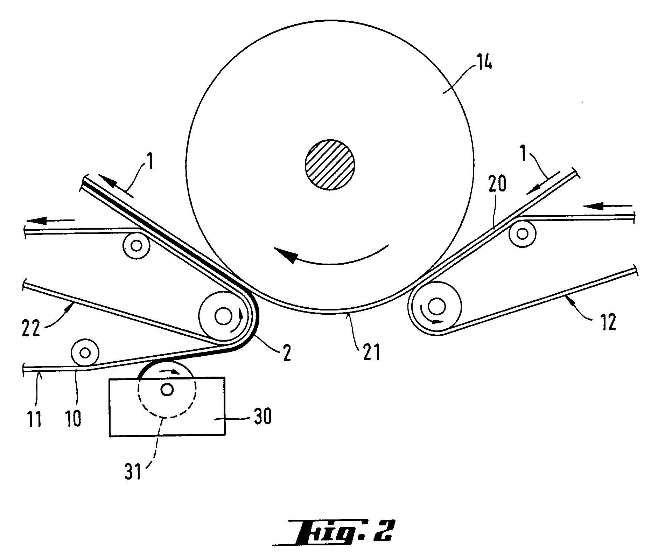

As will be obvious for those skilled in the art, the shape, the depth, and density of individual engravings on the rotating transport roll surface in addition to the overall pattern of the engravings will be a critical parameter for the total amount of adhesive and basis weight supplied to first surface (11) of first material (10). Examples of the particular shape of engravings which have been found useful in the context of adhesive application for disposable absorbent articles such as sanitary napkins and pantiliners are shown in figures 4 through 15. The engravings are shown as a top plan view onto the rotating transport roll. For orientation a line is shown designated by reference numeral 3 which is parallel to the rotation axis of the rotating transport roll (31).

As can be seen in figures 1, 2 or 3 this direction is typically perpendicular to the machine direction defined by the production path of the first material.

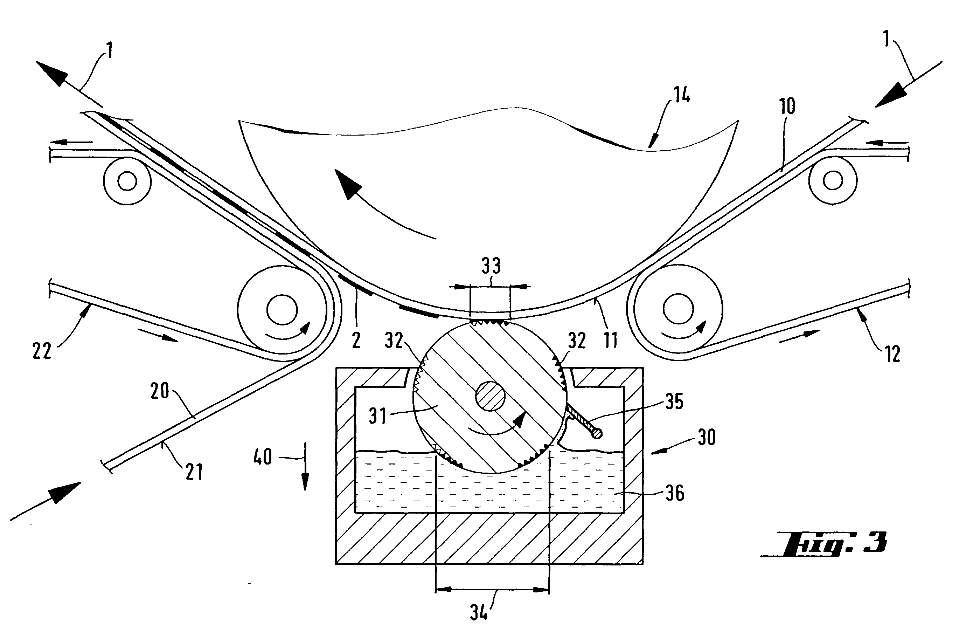

Figure 4 shows a square pyramidal shape with a depression angle of about 45° which can be seen in the cross-section shown in figure 5. Other angles such as 30°, 15° or steeper engravings at 60° or 75° can also be used. Figure 6 is similar to the square pyramidal engraving of figure 4, however, with a truncated central part of the pyramids which is shown in figure 7 in a cross-sectional view.

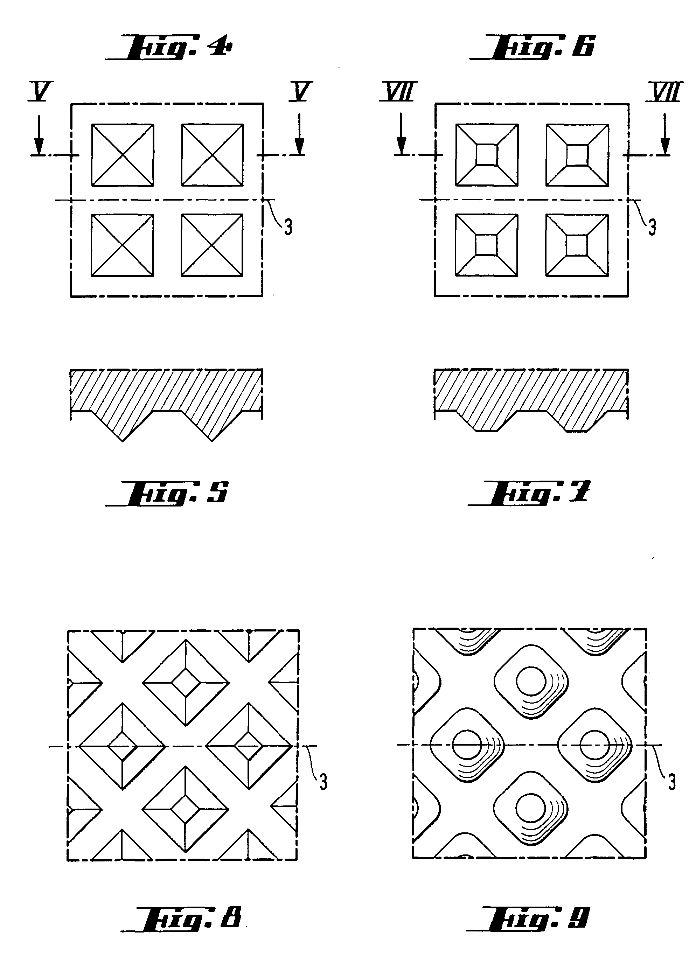

The direction of the individual engravings can of course alter relative to the rotation axes direction (3). This is seen when comparing figure 6 and figure 8 which shows the same truncated pyramid square engravings, however, with a change of their position relative to direction (3). Figure 9 shows a similar truncated engraving as figure 8, however, with the corners of the squares rounded. Figure 10 shows the same engraving as figure 9, however, with a different direction of the individual engravings. A cross-sectional representation of the engraving of figure 10 is shown in figure 11.

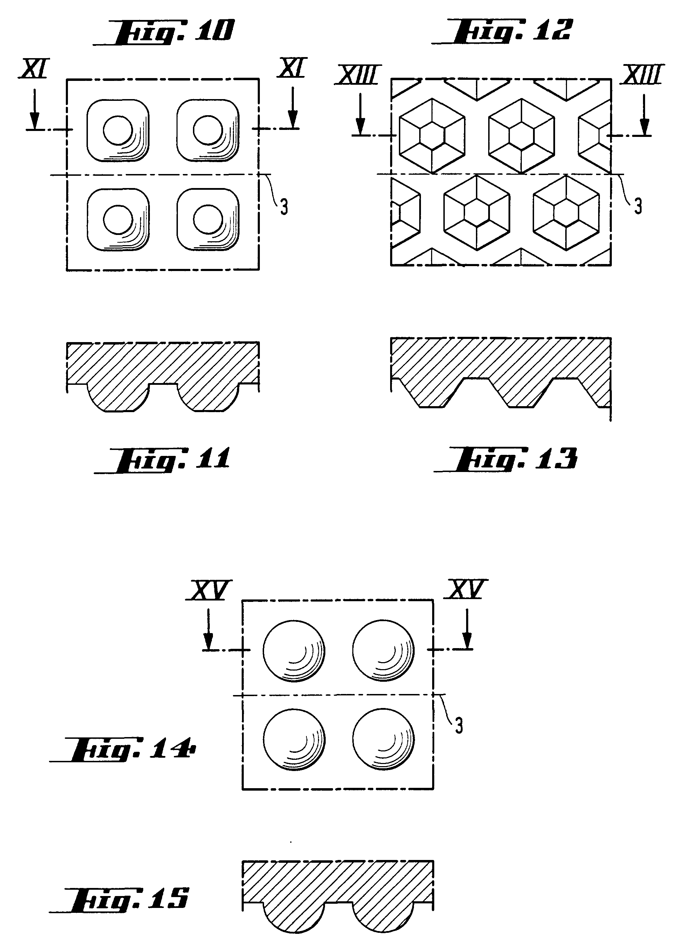

Figure 12 shows another alternative embodiment of the engraving in a truncated hexagonal pyramid. A cross-sectional representation of this engraving is shown in figure 13. Figure 14 and 15 show a round spherical engraving which is also an alternative.

The dimensions and density of these engravings can vary depending on the desired result of the adhesive printing step of the present invention. In the following preferable ranges for the engravings are given.

Each individual engraving can have a depth of from 0.01 mm for very small amounts of adhesive to be provided to 0.8 mm for the transport of large quantities of adhesive. An engraving size of 0.01 mm up to 2.5 mm as the side length for rectangular engravings or as the diameter for circular engraving has been found useful. For engravings which are neither square nor circular similar dimensions as those for square or circular engravings will be easily definable for those skilled in the art. The dimensions are of course taken on the surface of the rotating transport roll. A distance between engravings of 0.5 times to 50 times their depth has been found useful to provide an approximately even adhesive quantity distribution across the total engraved pattern.

The number of engravings per area depends on the individual engraving size. With increasing number of engravings and reducing size of individual engraving a more even adhesive delivery across the whole adhesive area will be achieved. Ultimately, a full surface engraving which parallels the adhesive area can be utilised.

An example of an engraving which has been successfully used in the context of the present invention is the engraving as shown in figure 6 with a depth of 0.067 mm, a size of 0.2 mm times 0.2 mm and 567 engravings per cm². These engravings were used to provide a panty fastening adhesive onto a polyethylene backsheet of a sanitary napkin according to the general description above. The panty fastening adhesive area followed the peripheral outline of the sanitary napkin in a so-called dogbone-shape in the narrowest part of the pad at a distance of approximately 4 mm.

Four alternative panty fastening adhesives were successfully tested at different temperatures. They were Savare LA203 which was applied at 130°C, Savare LA303 which was applied at 110°C, Fuller D3964ZP which was applied at 140°C and Coramelt 867 which was applied at 140°C. All application tests were conducted at surface speeds of up to 100 m/min. The second material providing the second surface was an usual siliconized release paper. Savare adhesives are available from Savare I.C. of Milan in Italy, Fuller adhesives are available from H.B. Fuller of Lueneburg in Germany and Coramelt adhesive is available from Koemmerling of Pirmasens in Germany.

## Claims

1. A process for providing a disposable absorbent article, said article as delivered to the user comprising a first material (10) which comprises a first surface (11) and comprising a second material (20) which comprises a second surface, said first material being joined to said second material by adhering at least part of said first and said second surface to each other, said process comprising the steps of
a) providing and conveying said first material (10) in a machine direction (1),
b) providing and conveying said second material (20),
c) providing an adhesive area onto at least part of said first surface (11),
d) interfacing said adhesive area on said first surface (11) with said second surface in order to join said first material (10) to said second material (20),
said process being characterized in that
an adhesive (2) is applied to said first surface (11) by roll printing, wherein said adhesive has an application temperature of 80°C or above and is delivered at or above said application temperature and is maintained at or above said application temperature while being transported by the rotating roll (31).

2. A process according to claim 1 wherein said roll printing comprises a rotating transport roll (31) for transporting said adhesive (2) said roll receiving said adhesive (2) in a receiving portion of its rotation path (34) said receiving portion (34) being within a bath (36) of said adhesive, said roll (31) transporting said adhesive from said receiving portion of its rotation path (34) by rotating to a delivering portion of its rotation path (33) and said roll (31) delivering said adhesive to said first surface (11) in said delivering portion of its rotation path (33).

3. A process according to any of the preceding claims wherein said roll printing provides said adhesive (2) to said first surface (11) from below, below being defined in a gravitational sence.

4. A process according to any of the preceding claims wherein said adhesive area has a peripheral edge comprising parts which are neither parallel nor perpendicular to said machine direction (1).

5. A process according to claim 4 wherein said parts of said peripheral edge of said adhesive area are non-linear.

6. A process according to any of the preceding claims wherein said first surface (11) of said first material (10) has a peripheral edge, said adhesive area has a peripheral edge and said peripheral edge of said adhesive area at least partially coincides with said peripheral edge of said first surface of said first material.

7. A process according to claim 6 wherein said peripheral edges of said adhesive area fully coincide with said peripheral edge of said first surface (11) of said first material (10).

8. A process according to any of the preceding claims wherein said adhesive area provides a panty fastening adhesive.

## Patentansprüche

1. Verfahren zur Schaffung eines aufsaugenden Wegwerfartikels, wobei der Artikel, wie er an den Anwender ausgeliefert wird, ein erstes Material (10) umfaßt, das eine erste Oberfläche (11) aufweist, und wobei er ein zweites Material (20) umfaßt, das eine zweite Oberfläche aufweist, wobei das erste Material mit dem zweiten Material verbunden wird durch Verkleben von zumindest einem Bereich der ersten und zweiten Oberfläche miteinander, wobei der Prozeß die Schritte umfaßt:
a) Bereitstellung und Förderung des ersten Materials (10) in einer Maschinenrichtung (1);
b) Bereitstellung und Förderung des zweiten Materials (20);
c) Auftrag einer Klebstofffläche auf zumindest einem Teil der ersten Oberfläche (11);
d) In-Kontakt-bringen der Klebstofffläche auf der ersten Oberfläche (11) mit der zweiten Oberfläche, um das erste Material (10) mit dem zweiten Material (20) zu verbinden,
wobei das Verfahren **dadurch gekennzeichnet** ist, daß
ein Klebstoff (2) auf die erste Oberfläche (11) durch Rollendruck aufgetragen wird, wobei der Klebstoff eine Auftragstemperatur von 80° C oder mehr aufweist und auf der oder über der Auftragstemperatur abgegeben wird und auf der oder über der Einsatztemperatur gehalten wird, während er durch die rotierende Rolle (31) gefördert wird.

2. Ein Verfahren nach Anspruch 1, wobei der Rollendruck eine rotierende Transportrolle (31) für den Transport des Klebstoffs (2) umfaßt, wobei die Rolle den Klebstoff (2) in einem Aufnahmeabschnitt ihrer Rotationsbahn (34) erhält, wobei der Aufnahmeabschnitt (34) sich in einem Bad (36) des Klebstoffs befindet, wobei die Rolle (31) den Klebstoff von dem Aufnahmeabschnitt ihrer Rotationsbahn (34) durch Rotation zu einem Abgabeabschnitt ihrer Rotationsbahn (33) fördert und wobei die Rolle (31) den Klebstoff an die erste Oberfläche (11) in dem Abgabeabschnitt ihrer Rotationsbahn (33) übergibt.

3. Verfahren nach einem der vorherigen Ansprüche, wobei der Rollendruck den Klebstoff (2) auf die erste Oberfläche (11) von unten aufträgt, wobei unten in einem Schwerkraftssinn definiert ist.

4. Verfahren nach einem der vorherigen Ansprüche, wobei die Klebstofffläche einen Umfangsrand mit Abschnitten umfaßt, die weder parallel noch senkrecht zu der Maschinenrichtung (1) sind.

5. Verfahren nach Anspruch 4, wobei die Abschnitte des Umfangsrands der Klebstofffläche nicht linear sind.

6. Verfahren nach einem der vorherigen Ansprüche, wobei die erste Oberfläche (11) des ersten Materials (10) einen Umfangsrand aufweist, die Klebstofffläche einen Umfangsrand umfaßt und der Umfangsrand der Klebstofffläche zumindest teilweise mit dem Umfangsrand der ersten Oberfläche des ersten Materials zusammenfällt.

7. Verfahren nach Anspruch 6, wobei der Umfangsrand der Klebstofffläche vollständig mit dem Umfangsrand der ersten Oberfläche (11) des ersten Materials (10) zusammenfällt.

8. Verfahren nach einem der vorherigen Ansprüche, wobei die Klebstofffläche einen Slipbefestigungsklebstoff vorsieht.

## Revendications

1. Procédé pour fournir un article absorbant jetable, ledit article, lorsqu'il est transmis à l'utilisateur, comprenant un premier matériau (10) qui présente une première surface (11), et un deuxième matériau (20) qui présente une deuxième surface, ledit premier matériau étant réuni audit deuxième matériau en faisant adhérer l'une à l'autre au moins une partie de ladite première surface et une partie de ladite deuxième surface, ledit procédé comprenant les étapes consistant à
a) fournir et transporter ledit premier matériau (10) dans le sens machine (1),
b) fournir et transporter ledit deuxième matériau (20),
c) fournir une zone adhésive sur au moins une partie de ladite première surface (11),
d) mettre en interface ladite zone adhésive de ladite première surface (11) avec ladite deuxième surface afin de réunir ledit premier matériau (10) audit deuxième matériau (20),
ledit procédé étant caractérisé
en ce qu'un adhésif (2) est appliqué sur ladite première surface (11) par impression au rouleau, dans lequel ledit adhésif a une température d'application de 80°C ou plus, et est transmis à la ladite température d'application ou à une température supérieure, et est maintenu à ladite température d'application ou à une température supérieure tout en étant transporté par le rouleau rotatif (31).

2. Procédé selon la revendication 1, dans lequel ladite impression au rouleau comprend un rouleau rotatif de transport (31) destiné à transporter ledit adhésif (2), ledit rouleau recevant ledit adhésif (2) dans une partie de réception de son trajet de rotation (34), ladite partie de réception (34) étant à l'intérieur d'un bain (36) dudit adhésif, ledit rouleau (31) transportant par rotation ledit adhésif de ladite partie de réception de son trajet de rotation (34) à une partie de transmission de son trajet de rotation (33), et ledit rouleau (31) transmettant ledit adhésif à ladite première surface (11) dans ladite partie de transmission de son trajet de rotation (33).

3. Procédé selon l'une quelconque des revendications précédentes, dans lequel ladite impression au rouleau fournit ledit adhésif (2) à ladite première surface (11) par-dessous, dessous étant défini dans un sens gravitationnel.

4. Procédé selon l'une quelconque des revendications précédentes, dans lequel ladite zone adhésive présente un bord périphérique comprenant des parties qui ne sont ni parallèles ni perpendiculaires audit sens machine (1).

5. Procédé selon la revendication 4, dans lequel lesdites parties dudit bord périphérique de ladite zone adhésive ne sont pas linéaires.

6. Procédé selon l'une quelconque des revendications précédentes, dans lequel ladite première surface (11) dudit premier matériau (10) présente un bord périphérique, ladite zone adhésive présente un bord périphérique et ledit bord périphérique de ladite zone adhésive coïncide au moins partiellement avec ledit bord périphérique de ladite première surface dudit premier matériau.

7. Procédé selon la revendication 6, dans lequel lesdits bords périphériques de ladite zone adhésive coïncident complètement avec ledit bord périphérique de ladite première surface (11) dudit premier matériau (10).

8. Procédé selon l'une quelconque des revendications précédentes, dans lequel ladite zone adhésive fournit un adhésif de fixation de culotte.
